Europäisches Patentamt

**European Patent Office**

Office européen des brevets

(11) Veröffentlichungsnummer : **0 068 458**
**B1**

(12) **EUROPÄISCHE PATENTSCHRIFT**

(45) Veröffentlichungstag der Patentschrift :
13.03.85

(21) Anmeldenummer : **82105632.2**

(22) Anmeldetag : **25.06.82**

(51) Int. Cl.⁴ : **C 02 F   1/72,** G 05 D 21/02

(54) **Verfahren zur Behandlung · von kontinuierlichen Abwasserströmen mit wechselnden Gehalten verschiedener oxidierbarer Inhaltsstoffe mit Wasserstoffperoxid.**

(30) Priorität : **29.06.81 DE 3125452**

(43) Veröffentlichungstag der Anmeldung :
**05.01.83 Patentblatt 83/01**

(45) Bekanntmachung des Hinweises auf die Patenterteilung : **13.03.85 Patentblatt 85/11**

(84) Benannte Vertragsstaaten :
**AT BE CH DE FR GB IT LI NL SE**

(56) Entgegenhaltungen :
**EP-A- 0 009 580
DE-A- 2 007 727
DE-A- 2 009 826
FR-A- 2 219 120
POLLUTION ENGINEERING, Band 8, Nr. 3, März
1976, Seiten 43-45, Greenwich, USA**

(73) Patentinhaber : **Degussa Aktiengesellschaft
Weissfrauenstrasse 9
D-6000 Frankfurt am Main 1 (DE)**

(72) Erfinder : **Knorre, Helmut, Dr. Dipl.-Chem.
Ratleikstrasse 8c
D-6453 Seligenstadt (DE)**
Erfinder : **Fischer, Joachim
Buchbergstrasse 3
D-6458 Rodenbach (DE)**
Erfinder : **Stützel, Klaus
Nordring 74
D-6000 Frankfurt 60 (DE)**

**Beschreibung**

Unter den heutigen Aspekten des Umweltschutzes gewinnt die Verwendung von Wasserstoffperoxid als Mittel zur oxydativen Behandlung von Abwässern zunehmende Bedeutung, da Wasserstoffperoxid selbst nicht zur zusätzlichen Aufsalzung des Abwassers beiträgt, sondern zu Wasser und Sauerstoff aufspaltet. Eine wesentliche Voraussetzung ist jedoch, dass die erforderliche Zusatzmenge meßtechnisch erfasst und gesteuert werden kann.

In besonderem Maße gilt diese Forderung für die Behandlung von kontinuierlichen Abwasserströmen, die wechselnde Mengen unterschiedlich oxidierbarer Schadstoffe enthalten und nur eine relativ kurzzeitige Behandlung gestatten. In diesen Fällen ist der Erfolg einer solchen Abwasserbehandlung mit Wasserstoffperoxid in hohem Maße davon abhängig, wie schnell und zuverlässig man den gesamten Oxidationsmittelbedarf beim Einfließen des Abwasserstromes in die Behandlungsanlage zu erfassen vermag. Als Gesamtoxidationsmittel-Bedarf ist dabei diejenige Menge an Aktivsauerstoff bzw. $H_2O_2$ zu verstehen, die benötigt wird, um die oxidierbaren Schadstoffe im Abwasser unter den gewählten Reaktionsbedingungen in die jeweils möglichen Oxidationsprodukte überführen zu können.

In der Fachliteratur sind nur wenige Anhaltspunkte dafür zu finden, wie der gesamte Oxidationsmittelbedarf eines Abwassers meßtechnisch erfasst werden kann. Der Grund hierfür ist der, dass die meisten im Abwasser vorkommenden Schadstoffe nur in geringem und sehr unterschiedlichem Maße zur Bildung eines definierten Redoxpotentials, das z. B. mit einer Platin-Bezugselektrodenkette gemessen werden könnte, beitragen. Bei den oxidativen Abwasserbehandlungs-Verfahren wird deshalb überwiegend so gearbeitet, dass man unter Verfolgung des Redoxpotentials dem Abwasser sukzessive solange Oxidationsmittel zusetzt, bis sich durch den Überschuss ein gleichbleibendes Endpotential eingestellt hat. Dieses Verfahren ist jedoch in den meisten Fällen nur für eine batchweise Behandlung von Abwässern brauchbar, da die Oxidation der verschiedenen Schadstoffe mit $H_2O_2$ mehr oder minder lange Reaktionszeiten erfordert und sich somit ein gleichbleibendes Endpotential auch in Gegenwart von überschüssigem $H_2O_2$ nicht sofort, sondern erst dann einstellt, wenn die Oxidationsreaktionen zu Ende gekommen sind.

So z. B. wird in der DE-C-23 52 856 ein Verfahren zur Entgiftung cyanidischer Abwässer mit $H_2O_2$ beschrieben, das es erlaubt, den Entgiftungsvorgang und die Zugabe des $H_2O_2$ durch Messung des Redoxpotentials mit einer Silberelektrode und einer beliebigen. Bezugselektrode, vorzugsweise einer Thalamid-Elektrode, zu verfolgen und zu regeln. Dieses Verfahren ist jedoch in den meisten Fällen nur für eine batchweise Behandlung von Abwässern einsetzbar, da die Messung des Redoxpotentials zwar den freien Cyanidgehalt im Abwasser anzeigt, aber dann keine Schlüsse auf den Gesamtoxidationsmittel-Bedarf gestattet, wenn ausser freien Cyaniden noch andere Verbraucher von Wasserstoffperoxid im Abwasser enthalten sind.

Die einmalige Zugabe einer der Anzeige des freien Cyanidgehaltes entsprechenden Menge Oxidationsmittel zu Beginn der Abwasserbehandlung würde demnach zu keinem ausreichenden Entgiftungsergebnis führen, weil die anderen $H_2O_2$-Verbraucher einen Teil des für die Cyanid-Oxidation benötigten Wasserstoffperoxids für sich beanspruchen. Nur durch mehrfaches Nachdosieren von Oxidationsmittel in gewissen Zeitintervallen kann in diesen Fällen sowohl der gewünschte Abbau an Cyanid, als auch an anderen oxidierbaren Inhaltsstoffen herbeigeführt werden. Eine solche schrittweise Annäherung an das Reaktionsziel, wie sie auf Grund der für die Oxidation des Cyanids benötigten Reaktionszeit erforderlich ist, lässt sich jedoch für eine kontinuierliche Behandlung grosser Abwasserströme meist nicht anwenden, weil die Behandlungsanlage überdimensional gross gestaltet werden müsste, um den ausreichenden Behandlungszeitraum zu gewährleisten.

In der BE-A-883 046 wird ein Verfahren zur Messung der Konzentration einer bestimmten gelösten Komponente mit redox- oder ionensensitiven Elektroden zum Zwecke der Steuerung bzw. Regelung chemischer Prozesse u. a. bei der Abwasserbehandlung angegeben, welches dadurch gekennzeichnet ist, daß man in einem Seitenstrom des Systems durch Zugabe von überschüssigem Reaktionsmittel den maximal erreichbaren Potentialwert feststellt und die Abweichung vom Ausgangspotential als Maßgröße für den Schadstoffgehalt wertet. Auf diese Weise kann z. B. der freie Cyanidgehalt in einem kontinuierlichen Abwasserstrom schnell und sicher ermittelt und die für die Oxidation erforderliche Menge an Wasserstoffperoxid gezielt zudosiert werden. Das nach der BE-A-883 046 beschriebene Verfahren versagt jedoch, wenn im Abwasser verschiedene oxidierbare Schadstoffe in wechselnden Konzentrationen nebeneinander vorliegen und in unterschiedlichem Maße zur Bildung des Redox-Potentials beitragen. So z. B. wird bei der Messung des Cyanidpotentials mit einer Silberelektrode und einer Thalamid-Bezugselektrode unter den Bedingungen des in DE-C-2 352 856 beschriebenen Verfahrens durch 1 mg $S^{2-}$/l ungefähr der gleiche Redox-Meßwert von ca. 350 mV erzeugt wie durch 50 mg $CN^-$/l. Andere oxidierbare Stoffe wie z. B. Sulfit werden dagegen durch dieses Redox-System praktisch überhaupt nicht erfaßt.

Der Erfindung lag deshalb die Aufgabe zugrunde, ein Meßsystem zu entwickeln, das eine schnelle und zuverlässige Erfassung des Oxidationsmittelbedarfs ermöglicht, der für die Absenkung der Schadstoffkonzentration eines Abwassers unter einen gewünschten Grenzwert bei der kontinuierlichen oxidativen Behandlung des Abwassers unter bestimmten pH- und Temperaturbedingungen erforderlich ist.

# 0 068 458

Es wurde nun gefunden, daß man diese Aufgabe der Behandlung kontinuierlicher Abwasserströme mit wechselnden Gehalten verschiedener oxidierbarer Inhaltsstoffe, insbesondere Cyaniden, Sulfiden, Sulfiten, Thiosulfaten, Thiocyanaten und/oder auch organischen Substanzen mit Wasserstoffperoxid zum Zwecke der Entgiftung und Absenkung des chemischen und biologischen Sauerstoffbedarfs lösen kann, wenn man die Zudosierung des $H_2O_2$ zum kontinuierlichen Abwasserstrom (Hauptstrom) durch Ermittlung des Oxidationsmittelbedarfs in einem parallel davon laufend abgezweigten kleinen Teilstrom (Meßstrom) regelt, indem man im Teilstrom (Meßstrom)

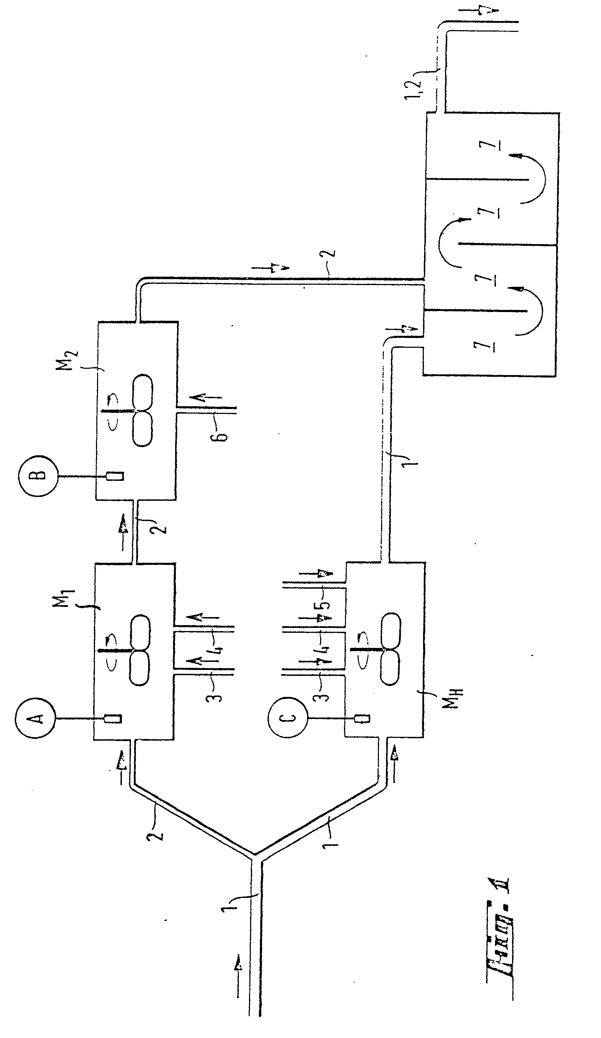

a) den pH-Wert des Abwassers durch Zudosieren von Lauge oder Säure kontinuierlich auf einen konstanten Wert zwischen pH 3 und 12 einreguliert

b) das Redoxpotential in an sich bekannter Weise mit einer Platin-, Silber-, Gold-, amalgamierter Silberelektrode oder ionensensitiven Elektrode und einer beliebigen Referenzelektrode kontinuierlich mißt und so lange eine wäßrige Lösung eines unter vergleichbaren Bedingungen schneller als $H_2O_2$ wirkenden Meß-Oxidationsmittels, vorzugsweise von Hypochlorit Peroxomonosulfat, Peroxodisulfat, Permanganat oder Ozon zudosiert, bis der gewünschte, dem angestrebten Schadstoffabbau entsprechende Redoxwert erreicht ist, und immer dann, wenn im Teilstrom (Meßstrom) das Meß-Oxidationsmittel zudosiert wird, proportionale Mengen an $H_2O_2$ und Hilfschemikalien, z. B. zur pH-Regulierung in den kontinuierlichen Abwasserstrom (Hauptstrom) gleichzeitig zugibt. Die Erfindung wird anhand Abb. 1 wie folgt näher erläutert :

Vom Abwasserstrom 1 (Hauptstrom) wird kontinuierlich eine zeitlich konstante Menge Abwasser abgezweigt und als sog. Meßstrom 2 separat durch zwei Meßgefäße M 1 und M 2 mit Rühr- bzw. Mischeinrichtungen geleitet. In Meßgefäß 2 werden durch eine pH- und eine Redox-Meßeinrichtung der pH- und Redoxwert des Abwassers kontinuierlich gemessen. In Meßgefäß M 1 wird durch Zudosieren von Säure oder Lauge der für die Messung des Redox-Potentials erforderliche pH-Wert kontinuierlich eingestellt und außerdem immer dann das für den Meßstrom vorgesehene Meß-Oxidationsmittel zugesetzt, wenn der im Gefäß M 2 gemessene Redoxwert vom gewünschten Redox-Sollwert abweicht.

Synchron zur Zudosierung des Meßoxidationsmittels in den Meßstrom 2 wird eine den Mengenproportionen von Meßstrom zu Abwasserstrom (Hauptstrom) proportionale Menge $H_2O_2$ in den Abwasserstrom 1 (Hauptstrom) zudosiert. Außerdem wird im Abwasserstrom 1 (Hauptstrom) der für die Abwasserbehandlung erforderliche pH-Wert einreguliert, sobald das Meßoxidationsmittel in den Meßstrom 2 bzw. $H_2O_2$ in den Abwasserstrom 1 (Hauptstrom) zudosiert wird.

Zur exakten und schnellen Erfassung des $H_2O_2$-Bedarfs, der für die Absenkung der Schadstoffkonzentrationen auf einen gewünschten Grenzwert erforderlich ist, mußte ein Meßoxidationsmittel ausgewählt werden, das einerseits praktisch augenblicklich mit den Schadstoffen im Abwasser reagiert und andererseits Oxidationsleistungen erzielt, wie sie bei der Dosierung von $H_2O_2$ in den Abwasserstrom 1 (Hauptstrom) innerhalb der zur Verfügung stehenden Behandlungsdauer erreicht werden können. Wie oben gesagt, haben sich unter diesen Bedingungen als geeignete Meßoxidationsmittel vor allem lösliche Salze der Peroxomonoschwefelsäure erwiesen. Aber auch andere starke Oxidationsmittel wie Salze der Peroxodischwefelsäure, Permanganate und Ozon sind anwendbar.

Die Zugabe des $H_2O_2$ sowie der Chemikalien zur pH-Regelung im Abwasserstrom 1 (Hauptstrom) nimmt man zweckmäßigerweise in einer Mischkammer MH vor, aus der man den Abwasserstrom 1 in ein oder mehrere hintereinander geschaltete Reaktionsbecken 7 leitet, in denen die Oxidation der verschiedenen Inhaltsstoffe nebeneinander ablaufen kann. Im Verlauf des Passierens dieser Reaktionsbecken kann evtl. auch eine Korrektur des pH-Wertes oder Nachdosierung von Oxidationsmittel vorgenommen werden.

Sofern bei der oxidativen Behandlung schwerlösliche Verbindungen gebildet werden, muß der Abwasserstrom anschließend entsprechend den Einrichtungen zur Klärung bzw. Abtrennung der Sedimente (nicht gezeigt) zugeführt werden, bevor er in den Vorfluter (nicht gezeigt) eingeleitet werden kann. Außerdem ist erforderlich, den pH-Wert auf den zulässigen Bereich des Vorfluters zu justieren, wenn er davon abweicht.

Auch der Meßstrom 2 tritt nach Durchlaufen der Meßgefäße M 1 und M 2 in den Abwasserstrom 1 (Hauptstrom) ein.

In Abbildung 1 sind die Meßgeräte durch die Buchstaben A, B, und C gekennzeichnet. Die Zudosierung von Säure, Lauge, $H_2O_2$ und Caroat in den Meßstrom 2 bzw. Abwasserstrom 1 (Hauptstrom) wird durch die Ziffern 3, 4, 5 und 6 angegeben.

Das erfindungsgemäße Verfahren hat sich vor allem bei der Entcyanisierung von Gichtgaswaschwässern bewährt. Solche Abwässer fallen bei Hochofenprozessen in Mengen von einigen Hundert bis über tausend $m^3/h$ an. Sie enthalten außer erheblichen Anteilen an Flugstaub wechselnde Mengen Alkalicyanide, Schwermetallcyanide, Sulfide, Sulfite, Thiocyanate und andere Schwefelverbindungen sowie Phenole und sonstige organische Verbindungen. Der Gehalt an leicht freisetzbarem Cyanid in diesen Waschwässern liegt normalerweise im Bereich von 0-10 mg $CN^-/l$ ; bei Störungen des Hochofenprozesses oder beim An- und Abfahren des Hochofens kann der Cyanidgehalt aber auch wesentlich höher (über 100 mg $CN^-/l$) ansteigen.

Für die Entcyanisierung solcher Abwässer stand bislang kein geeignetes Verfahren zur Verfügung : Die bekannten Verfahren der Chlorierung haben die ebenfalls bekannten Nachteile wie Bildung toxischer Chlorierungsprodukte, Arbeiten bei sehr hohem pH-Wert und dadurch Entstehen zusätzlicher

3

Neutralsalzmengen bei der nach der Entgiftung notwendigen Neutralisierung der Abwässer vor Eintritt in den Vorfluter, die sich zu den durch die Chlorierungsreagenzien entstehenden Neutralsalzen addieren sowie der bekannten Gefahren bei Anwesenheit von überschüssigem Chlor bzw. Hypochlorit, ganz abgesehen von den meß- und regeltechnischen Schwierigkeiten beim Arbeiten mit den genannten Stoffen.

Mit Wasserstoffperoxid dagegen treten keine toxischen oder aufsalzenden Produkte auf, und die Anwesenheit eines evtl. Überschusses schadet nicht, da sich Wasserstoffperoxid zu Wasser und Sauerstoff zersetzt. Die Cyanidoxidation mit Wasserstoffperoxid kann außerdem bei niedrigerem pH-Wert, vorzugsweise bei pH 10, ausgeführt werden ; deshalb benötigt man weniger Chemikalien für die Alkalisierung und Rückneutralisierung des Abwassers und erzeugt auch weniger Neutralsalz. Gleichzeitig werden durch Wasserstoffperoxid Schwefelverbindungen niederer Oxidationsstufe zu elementarem Schwefel oder Sulfat oxidiert und Thiocyanate in Cyanate übergeführt. In Gegenwart von metallischem Eisen oder Kupfer oder Eisen-III-Ionen werden auch Phenole durch $H_2O_2$ oxidativ abgebaut, wobei in der Hauptsache Dicarbonsäuren entstehen. Teilweise erfolgt die Oxidation organischer Verbindungen bis zu $CO_2$, so daß im allgemeinen eine deutliche CSB-Absenkung ohne zusätzliche Aufsalzung durch $H_2O_2$ bewirkt wird. Auch Aldehyde werden durch $H_2O_2$ oxidiert.

Vorversuche haben gezeigt, daß zur Absenkung des Cyanidgehaltes im Gichtgaswaschwasser eine in bezug auf das Cyanid deutlich erhöhte Menge an $H_2O_2$ erforderlich ist, weil andere oxidierbare Stoffe mit dem Wasserstoffperoxid teilweise schneller reagieren als das Cyanid. Eine solche Abwasserbehandlung mit $H_2O_2$ ist deshalb nur durchführbar, wenn der Zusatz an $H_2O_2$ meßtechnisch gesteuert und auf den jeweiligen Gesamtoxidationsmittelbedarf ausgerichtet werden kann. Dies wurde erst durch Anwendung des erfindungsgemäßen Verfahrens wie im nachfolgenden Beispiel 2 beschrieben, ermöglicht.

Ein weiteres Anwendungsgebiet für das erfindungsgemäße Verfahren ist z. B. die Behandlung von Abwässern aus den Reproduktionsabteilungen grafischer Betriebe und aus Filmkopierwerken, deren Schadstoffgehalt sowohl in den einzeln und getrennt geführten Abwasserströmen, als auch in Form von Abwassermischungen auf den gewünschten Grenzwert abgesenkt werden kann. Solche Abwässer enthalten neben anorganischen Schwefelverbindungen niederer Oxidationsstufe auch stets wechselnde Mengen organischer Verbindungen, die aufgrund ihres hohen Sauerstoffbedarfs (CSB, BSB) die biologischen Kläranlagen stark belasten. Mit Hilfe von Wasserstoffperoxid ist es möglich, solche Abwässer aus Fixier-, Entwicklungs-, Klärbad- und anderen Prozessen so zu behandeln, daß die biologische Belastung wesentlich geringer wird. So z. B. können durch die Behandlung mit $H_2O_2$ reduzierende Substanzen wie Thiosulfat und Sulfit, die in Fixier-, Entwicklungs- und Klärbädern vorkommen, letztlich bis zu Sulfat oxidiert werden. Gleichzeitig wird bei der oxidativen Behandlung mit $H_2O_2$ das gelöste Silber als schwerlösliches Gemisch von $Ag_2O$, $AgBr$ und $Ag_2S$ ausgefällt. Die Silberrückgewinnung aus dem Schlamm trägt so zur Kostendeckung der Abwasserbehandlung bei.

In konzentrierten Abwässern solcher Photoprozesse können die bei der Behandlung mit $H_2O_2$ auftretenden Veränderungen des pH-Wertes bzw. des Redoxpotentials oftmals zur Steuerung der oxidativen Abwasserbehandlung benützt werden, zumal diese meist batchweise vorgenommen werden kann. Wesentlich schwieriger gestaltet sich jedoch eine solche Abwasserbehandlung, wenn es sich um verdünnte Abwässer mit relativ geringen Konzentrationen an oxidierbaren Stoffen handelt und aufgrund der größeren Abwassermengen nicht batchweise, sondern kontinuierlich im Durchlauf behandelt werden muß.

Der Erfindung lag in diesem Falle die Aufgabe zugrunde, ein Meß- und Regelkonzept zu finden, mit dessen Hilfe es möglich ist, durch Zudosieren von $H_2O_2$ in einem kontinuierlichen Abwasserstrom den als Maßzahl für den Gehalt an oxidierbaren Stoffen dienenden Jodwert auf z. B. 10 mg Jod/l abzusenken.

Zur Durchführung des erfindungsgemäßen Verfahrens wird der pH-Wert so ausgewählt, daß die im Meß- und Abwasserhauptstrom eingesetzten Oxidationsmittel optimal wirken können. Dieser pH-Wert bzw. pH-Bereich kann im Abwasserhauptstrom und im Meßstrom verschieden sein.

So zeigte sich, daß bei der Oxidation cyanidischer Abwässer der pH im Haupt- und Meßstrom im Bereich von 6-12, vorzugsweise bei etwa 9-12 liegen kann. Der Wert von etwa 10 erwies sich als besonders günstig.

Als Redox-Meßketten wurden wahlweise Platin-, Gold- oder Silberelektroden bzw. eine ionensensitive Elektrode eingesetzt, die gegen beliebige Referenzelektroden, z. B. eine Thalamidelektrode, geschaltet wurden.

Beim Einsatz von Wasserstoffperoxid können auch Aktivatoren, z. B. die in der DE-C-2 352 856 beschriebenen Stoffe, von Vorteil sein.

Das Wasserstoffperoxid wird normalerweise in handelsüblichen Konzentrationen eingesetzt, z. B. in Lösungen mit 30-70 Gew.-% $H_2O_2$. Höhere oder niedrigere Konzentrationen sind möglich, aber nicht üblich.

Die als Meßoxidationsmittel verwendeten Stoffe wie z. B. Peroxomonosulfat, Peroxodisulfate, Kaliumpermanganat, Ozon oder Hypochlorit werden in Konzentrationen eingesetzt, die dem jeweiligen Oxidationsmittelbedarf des Abwassers angepaßt sind. Bei der Verwendung von Kaliumcaroat hat sich eine Konzentration von 45 g $KHSO_5$/l als zweckmäßig erwiesen.

Pro Mol Peroxomonosulfat werden 1-10 Mol $H_2O_2$ eingesetzt ; natürlich muß dabei das Mengenverhältnis von Liter Meßstrom zu Liter Hauptstrom berücksichtigt werden. Die geringen Mengen Oxydationsmittel, auch Hypochlorit, verursachen keine Schwierigkeiten.

Zur Entcyanisierung von Abwässern mit $H_2O_2$ kann ggf. auch eine kleine Menge Formaldehyd als Hilfsmittel eingesetzt werden, wodurch das Cyanid teilweise in Glykolsäure übergeführt wird. Der Formaldehyd wird in Form handelsüblicher wäßriger Lösungen eingesetzt, die bevorzugt 30-40 Gew.-% $H_2CO$ enthalten. Nach dem erfindungsgemäßen Verfahren sind Zusätze von 0,1-1 Mol $H_2CO$/Mol Peroxomonosulfat vorteilhaft, wobei wiederum das Mengenverhältnis von Liter Meßstrom zu Liter Hauptstrom berücksichtigt werden muß.

Der technische Fortschritt des erfindungsgemäßen Verfahrens besteht in der Möglichkeit, den für eine Absenkung der Konzentrationen verschiedener Schadstoffe erforderlichen Bedarf an $H_2O_2$ in kontinuierlichen Abwasserströmen schnell und kontinuierlich ermitteln sowie gleichzeitig die Dosierung des Wasserstoffperoxids sowie der Hilfschemikalien z. B. zur Einregulierung eines erforderlichen pH-Wertes in den Abwasserstrom vornehmen zu können, so daß sichergestellt ist, daß auch bei wechselnden Konzentrationen an Schadstoffen im Abwasser stets der gewünschte Abbau auf die geforderten Grenzkonzentrationen erfolgt. Das erfindungsgemäße Verfahren hat außerdem den Vorteil, daß die pH-Wert-Regulierung im Abwasserhauptstrom immer nur dann vorgenommen wird, wenn eine Behandlung mit Oxidationsmittel wie Wasserstoffperoxid notwendig ist. Dadurch werden Reaktionschemikalien eingespart sowie eine unnötige Aufsalzung des Abwasserstromes vermieden. Die Erfindung wird an folgenden Beispielen näher erläutert :

Beispiel 1

Einem kontinuierlichen Abwasserstrom aus einem Filmkopierwerk sollte eine dem wechselnden Gehalt an oxidierbaren Stoffen entsprechende Menge an $H_2O_2$ automatisch zugesetzt werden unter weitgehender Vermeidung von Über- und Unterdosierungen. Der hier als Maßzahl für den Gehalt an oxidierbaren Stoffen dienende Jodwert sollte auf max. 10 mg Jod pro Liter ausgeregelt werden.

In Vorversuchen wurde festgestellt, dass die Änderungen des Redoxpotentials bei den im unbehandelten Abwasser vorliegenden, relativ geringen Konzentrationen an oxidierbaren Stoffen (Jodwert 100-200 mg pro Liter) durch einen Zusatz von $H_2O_2$ nur klein und sich regelungstechnisch nicht verwerten liessen. Der in diesen Wasserproben vorliegende pH-Wert von 6-7 änderte sich durch die $H_2O_2$-Zugabe nicht wesentlich.

Es wurde aber festgestellt, dass auch bei diesen Abwässern deutliche, pH-abhängige Änderungen des Redoxpotentials (ca. 300-400 mV) auftreten, wenn dem Abwasser anstelle von $H_2O_2$ die Lösung zum Beispiel eines Alkalisalzes der Peroxomonoschwefelsäure (Caro'schen Säure) wie Kaliumcaroat, $KHSO_5$, zugesetzt wird. Diese Veränderung des Redoxpotentials beim Zusatz von Caroat lässt sich als Regelgrösse für die Behandlung des Abwassers mit $H_2O_2$ nützen, wenn der Caroat-Zusatz bei konstantem pH-Wert vorgenommen und die durch den Zusatz der sauer reagierenden Caroat-Lösung bewirkte pH-Änderung durch Natronlauge-Zusatz kompensiert wird. Zur Potentialmessung ist dabei ein Platin/Kalomel-Elektrodenpaar geeignet.

Zum Prinzip des erfindungsgemässen Verfahrens siehe Abbildung 1 :

Aus dem Betriebsabwasserstrom eines filmkopierwerkes (ca. 40 cbm pro Stunde) wurden für das vorliegende Beispiel ein Meßstrom (50 Liter pro Stunde) und ein Hauptstrom mit 2 300 Liter pro Stunde abgezweigt. Der Meßstrom wurde durch zwei hintereinander geschaltete Rührzellen (M 1 und M 2) geleitet. In die Rührzelle M 1 wurde die Caroat-Lösung (150 g Kaliumcaroat pro Liter = 6,32 g Aktivsauerstoff pro Liter) und in die Rührzelle M 2 wurde 1 N-Natronlauge (40 g NaOH pro Liter) eindosiert. Die Rührzelle M 2 enthielt ausserdem eine pH-Einstabmeßkette und ein Platin/Kalomel-Redoxelektrodenpaar.

Mit Hilfe eines Meß- und Regelgerätes wurde die Dosierung von Natronlauge und Caroatlösung in den Meßstrom so geregelt, dass bei einem konstanten pH-Wert von 8 ein Redoxpotential von + 150 mV konstant gehalten wurde. (Unter diesen Bedingungen wurde mit Kaliumjodid-Stärkepapier stets ein kleiner Caroat-Überschuss im Meßstrom festgestellt).

Synchron zur Caroat-Dosierung im Meßstrom wurde dem Hauptstrom eine proportionale Menge $H_2O_2$ (50 Gewichtsprozent 281 g Aktivsauerstoff pro Liter) zugesetzt. Das mit $H_2O_2$ versetzte Abwasser durchströmte den Reaktor MH, der aus zwei hintereinandergeschalteten Reaktoren von je ca. 750 Liter Volumen bestand, von denen der erste Reaktor mit einem Rührer ausgestattet war. Die theoretische Verweilzeit betrug je ca. 20 Minuten pro Reaktor.

Durch Bestimmung der Jodwerte des mit $H_2O_2$ behandelten Abwassers im Hauptstrom wurde die $H_2O_2$-Dosierung so eingestellt, dass der Jodwert, der im unbehandelten Abwasser im Mittel 200 mg $J_2$ pro Liter betrug, bis auf 10 mg Jod pro Liter abgesenkt wurde.

Die für die Erzielung dieses Jodwertes erforderlichen Chemikalienmengen betrugen im Durchschnitt :

a) 400 ml 1 N-NaOH pro Stunde und 680 ml Caroat-Lösung (150 g pro Liter) pro Stunde für den Meßstrom (50 Liter pro Stunde)

b) 940 ml $H_2O_2$, 50 gewichtsprozentig, pro Stunde für den Hauptstrom (2,3 cbm pro Stunde).

5

Beispiel 2

An dem Hochofen eines Hüttenbetriebes zur Erzeugung von Roheisen fallen pro Stunde ca. 1 400-1 800 cbm Gichtgaswaschwasser an, das neben Eisen- und Zinkoxiden, Erz- und Schlacketeilchen, Calciumverbindungen etc. wechselnde Mengen gelöster oxidierbarer Stoffe, wir Sulfite, Sulfide, Phenole und auch Alkalicyanide in der Größenordnung von 0-10 mg CN⁻ pro Liter enthält. Die Gesamtanalyse eines solchen Abwassers ergab zum Beispiel folgende Inhaltsstoffe :

2,15 mg CN⁻/l Gesamtcyanid nach DIN 38 405 - D 13.1
1,95 mg CN⁻/l direkt argentometrisch bestimmbares Cyanid
16   ml absetzbare Stoffe pro Liter Abwasser
0,5  mg Cu/l, gesamt
0,3  mg Ni/l, gesamt
17   mg Zn/l, gesamt
168  mg Fe/l, gesamt
0,3  mg Cu/l, gelöst im Filtrat
0,2  mg Ni/l, gelöst im Filtrat
<0,05 mg Zn/l, gelöst im Filtrat
0,3  mg Fe/l, gelöst im Filtrat.

Der pH-Wert des Abwassers lag bei ca. 6. Die Permanganat-Zahl betrug 1 049 mg KMnO₄ pro Liter Abwasser entsprechend 265,6 mg O aktiv pro Liter bzw. 1 130 mg H₂O₂, (50 gewichtsprozentig) pro Liter bzw. 5 600 mg Kaliumcaroat, (mit 45 Prozent KHSO₅), pro Liter Abwasser.

Bei der vorübergehenden Stillsetzung des Hochofens zum Zwecke der Reparatur wurden im Gichtgaswaschwasser folgende maximalen Schadstoffgehalte festgestellt :

197   mg CN⁻/l direkt argentometrisch bestimmbares Cyanid
142   mg CN⁻/l leicht freisetzbares Cyanid nach DIN 38 405 - D 13.2
820   mg Fe/l, gesamt
7,6 mg Pb/l
87,2 mg Zn/l
39   mg S⁻⁻/l

Bislang wurden die Abwässer dieses Hochofens lediglich in einer Sedimentationsanlage (Bischoff-Becken) kontinuierlich geklärt und mit anderen Prozeßwässern vermischt in einen Fluß eingeleitet ; nur beim An- und Abfahren des Hochofens, d. h. bei höheren Cyanidgehalten im Gichtgaswaschwasser wurde eine Behandlung mit Hypochloritbleichlauge durchgeführt. Dieses Verfahren befriedigte aber auf Grund der oben angeführten Nebeneffekte nicht.

Im Rahmen der Untersuchung sämtlicher infrage kommenden Verfahren zur Entcyanisierung dieser Abwässer konnte nun auch die Behandlung mit Wasserstoffperoxid unter Anwendung der erfindungsgemässen Meß- und Regeltechnik erprobt werden. Es zeigte sich dabei, dass das leicht freisetzbare Cyanid vor allem dann — unter Nutzung der Verweilzeit im Sedimentationsbecken — schnell und sicher auf Werte unter 0,1 mg CN⁻ pro Liter (nach DIN 38 405 - D. 13.2) abgesenkt werden kann, wenn die durch redoxgesteuerte Zugabe von Kaliumcaroat in einem Zweigstrom (Meßstrom) ermittelte Menge H₂O₂ dem Abwasser bereits vor der Sedimentationsanlage im Sammelbecken zugegeben und gleichzeitig eine — bezogen auf die H₂O₂-Menge — unterstöchiometrische Menge an Formaldehyd zugegeben wird. Dadurch wird vor allem bei niedrigen Cyanidkonzentrationen eine teilweise Umwandlung in Glykolnitril bewirkt, das unter Einwirkung von H₂O₂ sehr rasch zu glykolsäure hydrolysiert und in Gegenwart von Calcium-Ionen weitgehend als schwerlösliches Calciumglykolat ausgefällt wird :

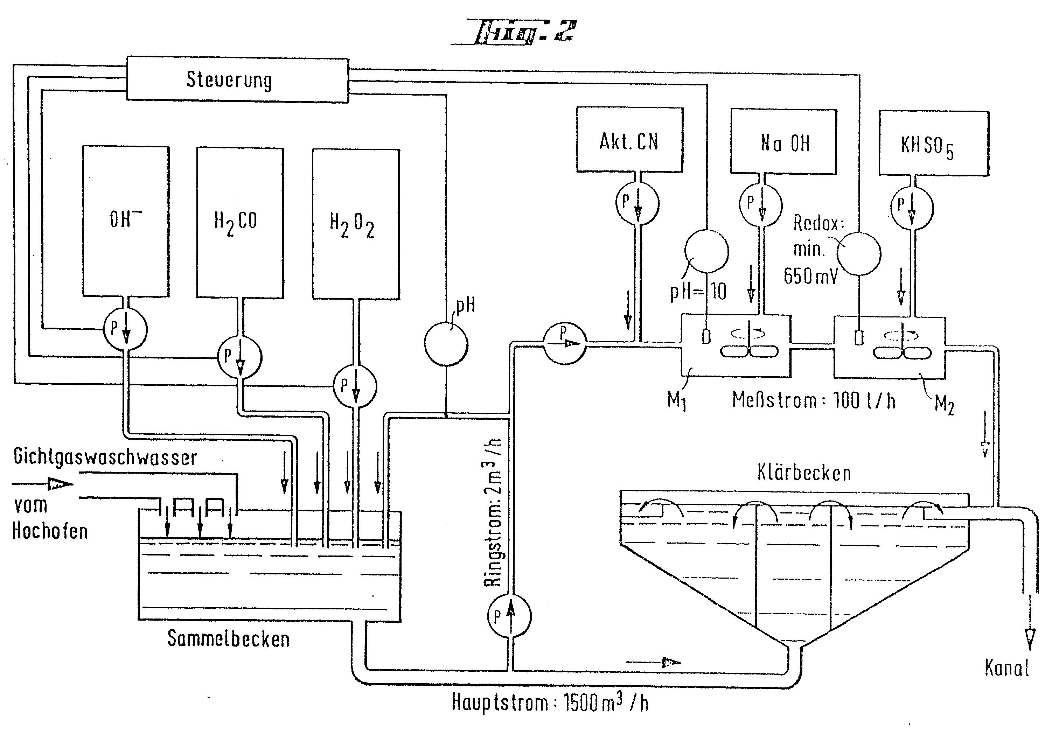

Das Prinzip der für die Entcyanisierung der Gichtgaswaschwässer mit H₂O₂ und H₂CO angewandten Meß- und Regeltechnik ist in Abbildung 2 dargestellt.

Zur Regelung der Chemikalien-Zugabe wird vom Gichtgaswaschwasser-Strom des Hochofens ein Teilstrom von ca. 2 cbm Abwasser pro Stunde abgezweigt und als Ringstrom wieder in das Sammelbecken zurückgeführt. Von diesem Ringstrom werden kontinuierlich genau 100 Liter Abwasser pro Stunde abgezogen und als Meßstrom durch zwei Mischgefäße M 1 und M 2 geleitet. Im Mischgefäß M 1 wird der pH-Wert des Abwassers mit einer Glaselektrodenkette kontinuierlich gemessen und durch automatisches Zudosieren von 10 %iger Natronlauge auf pH 10 ± 0,5 einreguliert. Im Mischgefäß M 2 wird das Redoxpotential des Abwassers mit einer Silber- und einer Thalamid-Bezugselektrode kontinuierlich gemessen und durch automatisches Zudosieren einer 10 %igen Caroat-Lösung mit 4,5 % KHSO₅ auf mindestens + 650 mV einreguliert. Dieses Potential entsprach dem gewünschten Entgiftungsgrad. Zur Messung dieses Potentials werden dem Meßstrom 2 pro Liter mindestens 0,001 mg AgNO₃ und 2,5 mg KJ kontinuierlich zudosiert. Die Leistung der Dosierpumpe für die Caroat-Lösung war dabei so bemessen, dass im Normalbetrieb des Hochofens Schadstoffgehalte von max. 10 mg CN⁻ pro Liter in Gegenwart der anderen oxidierbaren Stoffe durch die zugegebene Menge Caroat-Lösung praktisch augenblicklich auf Restgehalte < 0,1 mg CN⁻ pro Liter abgesenkt werden konnten (z. B. Dosierleistung 1 Liter 10 %ige

Caroat-Lösung pro Stunde). Die Pumpe zur Dosierung von Caroat-Lösung in den Meßstrom wurde über den Schaltpunkt der Redox-Meßeinrichtung angesteuert : Beim Unterschreiten des Schaltpunktes (= Soll-mV-Wert) wurde die Pumpe eingeschaltet, beim Überschreiten des gleichen Schaltpunktes wieder abgeschaltet.

Parallel zur Dosierung der Caroat-Lösung wurde beim Unterschreiten des Schaltpunktes des Redox-Meßverstärkers auch die Chemikalienzugabe in den Hauptstrom angesteuert. Über eine pH-Meßeinrichtung in der Ringstromleitung wurde der pH-Wert des Abwasserhauptstromes, der schon durch Kalk voralkalisiert wurde, durch Zudosieren von Natronlauge in das Sammelbecken auf pH 10 ± 0,5 einreguliert, sowie proportionale Mengen an 37 %iger Formalinlösung und 50 gewichtsprozentigem Wasserstoffperoxid zudosiert. Die Dosierleistungen der Chemikalienpumpen waren dabei so ausgelegt, dass im Normalbetrieb des Hochofens und einer Abwassermenge von 1 500 cbm pro Stunde pro liter Caroat-Lösung (in den Meßstrom) 620 Liter $H_2O_2$ (50 gewichtsprozentig) pro Stunde und 170 Liter $H_2O_2$ (37 gewichtsprozentig) sowie ca. 3 000 Liter NaOH (20 gewichtsprozentig) dosiert werden konnten.

Der effektive Chemikalienverbrauch lag im Normalbetrieb des Hochofens bei ca. 250 Liter $H_2O_2$ (50 gewichtsprozentig) pro Stunde und 70 Liter $H_2CO$ (37 gewichtsprozentig) pro Stunde : ausserdem wurden durchschnittlich 840 Liter NaOH (20 gewichtsprozentig) pro Stunde für die Nachalkalisierung benötigt. Eine Rückneutralisation des Abwassers nach der oxidativen Behandlung war nicht erforderlich ; der pH-Wert des behandelten Abwassers lag nach der Sedimentabscheidung stets unter 9.

Eine in regelmässigen Zeitabständen durchgeführte naßanalytische Untersuchung des Entgiftungsgrades nach DIN 38 405 — D 13.2 ergab, dass unter diesen Bedingungen der Gehalt an leicht freisetzbarem Cyanid im Abwasser stets auf Werte $< 0,1$ mg CN$^-$/l abgesenkt werden konnte. Die Behandlung entsprach somit voll den Anforderungen.

Beim Ansteigen der Schadstoffgehalte im Abwasser musste sowohl die Dosierleistung der Caroat-Pumpe, als auch die der Chemikalienpumpen für den Hauptstrom proportional erhölt werden. Das Signal dazu wurde dadurch gegeben, dass bei permanentem Betrieb der Chemikalien-Dosierpumpen das Redox-Sollpotential nicht mehr überschritten wurde.

Bei der Behandlung von Gichtgaswaschwasser aus Hochofen-Prozessen ist es ausserdem erforderlich, die Funktion der Meßelektroden durch eine Reinigung in regelmässigen Zeitintervallen (ca. 1 Stunde) vorzunehmen, da die Schadstoffe im Abwasser (vor allem die Erdalkali-Verbindungen und das $CO_2$) im alkalischen pH-Bereich zu einer starken Belagbildung führen. Eine solche Reinigung der Elektroden konnte sehr leicht dadurch bewirkt werden, indem in regelmässigen Zeitabständen eine kleine Menge halbkonzentrierter Salzsäure oder Salpetersäure in das Mischgefäss M 1 des Meßstromes dosiert wurde. Diese Dosierung wurde durch eine Zeitschaltuhr automatisch geregelt.

**Ansprüche**

1. Verfahren zur Behandlung kontinuierlicher Abwasserströme mit wechselnden Gehalten verschiedener oxidierbarer Inhaltsstoffe insbesondere Cyaniden, Sulfiden, Sulfiten, Thiosulfaten, Thiocyanaten und/oder auch organischen Substanzen mit Wasserstoffperoxid zum Zwecke der Entgiftung und Absenkung des chemischen und biologischen Sauerstoffbedarfs, dadurch gekennzeichnet, daß man die Zudosierung des $H_2O_2$ zum kontinuierlichen Abwasserstrom (Hauptstrom) durch Ermittlung des Oxidationsmittelbedarfs in einem parallel davon laufend abgezweigten kleinen Teilstrom (Meßstrom) regelt, indem man im Teilstrom (Meßstrom)

a) den pH-Wert des Abwassers durch Zudosieren von Lauge oder Säure kontinuierlich auf einen konstanten Wert zwischen pH 3 und 12 einreguliert,

b) das Redoxpotential in an sich bekannter Weise mit einer Platin-, Silber-, Gold-, amalgamierter Silberelektrode oder ionensensitiven Elektrode und einer beliebigen Referenzelektrode kontinuierlich mißt und so lange eine wäßrige Lösung eines unter vergleichbaren Bedingungen schneller als $H_2O_2$ wirkenden Meß-Oxidationsmittels vorzugsweise Hypochlorit, Peroxomonosulfat, Peroxodisulfat, Permanganat oder Ozon zudosiert, bis der gewünschte, dem angestrebten Schadstoffabbau entsprechende Redoxwert erreicht ist, und immer dann, wenn im Teilstrom (Meßstrom) das Meß-Oxidationsmittel zudosiert wird, proportionale Mengen an $H_2O_2$ und Hilfschemikalien z. B. zur pH-Regulierung in den kontinuierlichen Abwasserstrom (Hauptstrom) gleichzeitig zugibt.

2. Verfahren nach Anspruch 1, dadurch gekennzeichnet, daß man als Meßoxidationsmittel Salze der Peroxomonoschwefelsäure, vorzugsweise das Kaliumsalz der Peroxomonoschwefelsäure (Kaliumcaroat), verwendet.

3. Verfahren nach Anspruch 1 und 2, dadurch gekennzeichnet, daß man zur Absenkung des Cyanidgehaltes im Abwasser

a) den pH-Wert des Teilstroms (Meßstroms) kontinuierlich auf 10 ± 0,5 durch Zudosieren von Lauge oder Säure einreguliert,

b) den Cyanidgehalt im Teilstrom (Meßstrom) kontinuierlich durch Messung des cyanidspezifischen Redoxpotentials mit einem Silber-Thalamid-Elektrodenpaar entsprechend DE-C-2 352 856 verfolgt und eine wäßrige Lösung von Peroxomonosulfat zudosiert, bis ein Redox-Potential von mindestens + 650 mV angezeigt wird,

c) eine dem Peroxomonosulfat-Verbrauch im Teilstrom (Meßstrom) proportionale Menge Wasserstoffperoxid in den Abwasserstrom (Hauptstrom) zugibt und den pH-Wert während der Zugabe auf 9-12, vorzugsweise pH 10 ± 0,5 einreguliert.

4. Verfahren nach Anspruch 1-3, dadurch gekennzeichnet, daß man pro Mol Peroxomonosulfatverbrauch im Teilstrom (Meßstrom) eine dem Mengenverhältnis Liter Meßstrom zu Liter Hauptstrom proportionale Menge von 1-10 Mol $H_2O_2$ dem Abwasserstrom (Hauptstrom) zudosiert.

5. Verfahren nach Anspruch 1-4, dadurch gekennzeichnet, daß man pro Mol Peroxomonosulfat-Verbrauch im Teilstrom (Meßstrom) eine dem Mengenverhältnis Liter Meßstrom zu Liter Hauptstrom proportionale Menge von 0,1-1 Mol $H_2CO$ und 1-10 Mol $H_2O_2$ dem Abwasserstrom (Hauptstrom) zudosiert.

6. Verfahren nach Anspruch 1 und 2, dadurch gekennzeichnet, daß man bei Abwässern, die die Schwefelverbindungen Sulfid, Sulfit oder Thiosulfat enthalten und deren Gehalt an diesen Verbindungen durch den Summenparameter Jodwert (= Jodverbrauch des Abwassers in mg I/l, die Jodzahl) ausgedrückt werden kann, zur Absenkung dieses Jodwertes

a) den pH-Wert des Teilstromes (Meßstromes) kontinuierlich auf einen konstanten pH-Wert im Bereich von pH 3-12, vorzugsweise 6-10, durch Zudosieren von Säure oder Lauge einreguliert,

b) dem Teilstrom (Meßstrom) kontinuierlich eine wäßrige Lösung von Peroxomonosulfat zudosiert und deren Menge so bemißt, daß damit das Redoxpotential des Teilstromes kontinuierlich auf einen konstanten mV-Wert eingestellt wird, der für eine bestimmte, vorgegebene Absenkung des Jodwertes charakteristisch ist,

c) eine dem Peroxomonosulfatverbrauch im Teilstrom (Meßstrom) proportionale Menge Wasserstoffperoxid in den Abwasserstrom (Hauptstrom) zugibt und den pH-Wert während der Zugabe auf pH 3-12, vorzugsweise 6-10, einreguliert.

## Claims

1. Process for the treatment of continuous streams of effluent with variable contents of differing oxidizable constituents, in particular cyanides, sulphides, sulphites, thiosulphates, thiocyanates and/or organic substances with hydrogen peroxide for detoxicating and reducing the chemical and biological oxygen demand, characterised in that the addition of $H_2O_2$ to the continuous stream of effluent (main stream) is controlled by determining the demand of oxidizing agents in a small branched partial stream (measuring stream) running parallel therefrom, in which, in the partial stream (measuring stream)

a) the pH of the effluent is regulated continuously to a constant value of between pH 3 and 12 by addition of caustic alkali solution or acid,

b) the redox potential is measured continuously in known manner with a platinum-, silver-, gold-, amalgamated silver electrode or ion-sensitive electrode and an optional reference electrode, and an aqueous solution of a measuring oxidizing agent which acts more rapidly than $H_2O_2$ under comparable conditions, preferably hypochlorite, peroxomonosulphate, peroxodisulphate, permanganate or ozone is added until the desired redox value corresponding to the desired decomposition of noxious substances is achieved and, while the measuring oxidizing agent is being added to the partial stream (measuring stream), proportional quantities of $H_2O_2$ and auxiliary chemicals, for example for pH regulation, are simultaneously added to the continuous stream of effluent (main stream).

2. Process according to claim 1, characterised in that salts of peroxomonosulphuric acid, preferably the potassium salt of peroxomonosulphuric acid (potassium caroate) are used as measuring oxidizing agent.

3. Process according to claims 1 and 2, characterised in that, to reduce the cyanide content in the effluent,

a) the pH of the partial stream (measuring stream) is regulated continuously to 10 ± 0.5 by addition of caustic alkali solution or acid,

b) the cyanide content in the partial stream (measuring stream) is followed continuously by measuring the cyanide-specific redox potential with a silver thalamide electrode pair corresponding to DE-O-2 352 856 and an aqueous solution of peroxomonosulphate is added until a redox potential of at least + 650 mV is displayed,

c) a quantity of hydrogen peroxide proportional to the peroxomonosulphate consumption in the partial stream (measuring stream) is added to the effluent stream (main stream) and the pH is regulated to 9 to 12, preferably 10 ± 0.5 during addition.

4. Process according to claims 1 to 3, characterised in that a quantity of from 1 to 10 mol of $H_2O_2$ proportional to the ratio between litres of measuring stream and litres of main stream is added to the effluent stream (main stream) per mol of peroxomonosulphate in the partial stream (measuring stream).

5. Process according to claims 1 to 4, characterised in that a quantity of 0.1 to 1 mol of $H_2CO$ and 1 to 10 mol of $H_2O_2$ proportional to the ratio between litres of measuring stream and litres of main stream is added to the effluent stream (main stream) per mol of peroxomonosulphate consumption in the partial stream. (measuring stream).

6. Process according to claims 1 and 2, characterised in that, with effluents which contain the sulphur compounds sulphide, sulphite or thiosulphate and whose content of these compounds can be expressed by the sum total iodine value (= iodine consumption of the effluent in mg of I per I, the iodine number), to reduce this iodine value,

a) the pH of the partial stream (measuring stream) is regulated continuously to a constant pH in the range from 3 to 12, preferably 6 to 10 by addition of acid or caustic alkali solution,

b) an aqueous solution of peroxomonosulphate is added continuously to the partial stream (measuring stream) and the quantity thereof is calculated such that the redox potential of the partial stream can be adjusted continuously to a constant mV value which is characteristic of a specific, predetermined reduction of the iodine value,

c) a quantity of hydrogen peroxide proportional to the peroxomonosulphate consumption in the partial stream (measuring stream) is added to the effluent stream (main stream) and the pH is regulated to 3 to 12, preferably 6 to 10, during addition.

## Revendications

1. Procédé de traitement, par de l'eau oxygénée, d'écoulements continus d'eaux usées à teneurs variables en diverses matières oxydables, en particulier des cyanures, sulfures, sulfites, thiosulfates, thiocyanates et/ou aussi des substances organiques, afin de dépolluer et abaisser la consommation d'oxygène chimique et biologique, procédé caractérisé en ce que le dosage de $H_2O_2$ dans l'écoulement continu d'eau usée (courant principal) est réglé par évaluation de la consommation en agent oxydant dans un petit courant partiel (courant de mesure) dérivé du courant principal et parallèle à celui-ci, par le fait que dans le courant partiel (courant de mesure) :

a) le pH de l'eau usée est réglé, par addition de lessive alcaline ou d'acide, en continu, à une valeur constante entre pH 3 et 12,

b) le potentiel redox est mesuré en continu, de manière connue en soi, avec une électrode au platine, à l'argent, à l'or ou à l'argent amalgamé, ou une électrode sensible aux ions, et une électrode de référence quelconque, et on introduit une solution aqueuse d'un agent oxydant de mesure dont l'action est plus rapide que celle de $H_2O_2$ dans des conditions comparables, de préférence de l'hypochlorite, du peroxomonosulfate, du peroxodisulfate, du permanganate ou de l'ozone jusqu'à obtention de la valeur redox souhaitée, correspondant à la décomposition voulue de l'élément polluant, et l'on ajoute toujours, en même temps, même lorsque dans le courant partiel (courant de mesure), l'agent oxydant de mesure est introduit, des quantités proportionnelles d'$H_2O_2$ et de produits chimiques auxiliaires, par exemple pour la régulation du pH dans le courant continu d'eau usée (courant principal).

2. Procédé selon la revendication 1, caractérisé en ce que comme agent oxydant de mesure, on utilise des sels de l'acide peroxomonosulfurique, de préférence le sel de potassium de l'acide peroxomonosulfurique (« caroate » de potassium, sel de potassium de l'acide de caro).

3. Procédé selon les revendications 1 et 2, caractérisé en ce que pour abaisser la teneur en cyanures dans l'eau usée ;

a) le pH du courant partiel (courant de mesure) est ajusté en continu à $10 \pm 0,5$ par addition de lessive alcaline ou d'acide,

b) la teneur en cyanures du courant partiel (courant de mesure) est suivie en continu par mesure du potentiel redox spécifique aux cyanures, avec un couple d'électrodes argent-thalamide, selon DE-C-2 332 856 et on ajoute une solution aqueuse de peroxomonosulfate jusqu'à indication d'un potentiel redox d'au moins + 650 mV,

c) une quantité d'eau oxygénée proportionnelle à la consommation de peroxomonosulfate dans le courant partiel (courant de mesure) est introduite dans le courant d'eau usée (courant principal) et le pH est ajusté pendant cette introduction à 9-12, de préférence $10 \pm 0,5$.

4. Procédé selon les revendications 1 à 3, caractérisé en ce que pour la consommation d'une mole de peroxomonosulfate dans le courant partiel (courant de mesure), il est introduit dans le courant d'eau usée (courant principal) une quantité de 1 à 10 moles d'$H_2O_2$ proportionnelle au rapport en litres du courant partiel au courant principal.

5. Procédé selon les revendications 1 à 4, caractérisé en ce que pour la consommation d'une mole de peroxomonosulfate dans le courant partiel (courant de mesure), on ajoute une quantité de 0,1 à 1 mole de $H_2CO$ et de 1 à 10 moles d'$H_2O_2$ dans le courant d'eau usée (courant principal), proportionnellement au rapport litre du courant de mesure/litres de courant principal.

6. Procédé selon les revendications 1 et 2, caractérisé en ce que dans le cas d'eaux usées contenant des composés du soufre tels que les sulfures, sulfites ou thiosulfates, et dont la teneur en ces composés peut s'exprimer par le paramètre global de l'indice d'iode (consommation d'iode de l'eau usée en mg de I/1, indice d'iode), pour abaisser cet indice d'iode :

a) le pH du courant partiel (courant de mesure) est ajusté en continu à une valeur constante aux environs de pH 3-12, de préférence 6-10, par introduction d'acide ou de base,

b) on ajoute au courant partiel (courant de mesure), en continu, une solution aqueuse de peroxomonosulfate dont la quantité est mesurée de façon que le potentiel redox du courant partiel soit toujours ajusté à une valeur constante en mV, qui est caractéristique pour un abaissement déterminé prévu de l'indice d'iode,

c) on ajoute une quantité d'$H_2O_2$ proportionnelle à la consommation de peroxomonosulfate dans le courant partiel (courant de mesure) dans le courant d'eau usée (courant principal) et on ajuste le pH pendant cette introduction à une valeur de 3 à 12, de préférence 6 à 10.

Fig. 1

0 068 458

Fig. 2

Steuerung

OH⁻    H₂CO    H₂O₂    Akt. CN    Na OH    KHSO₅

Redox: min. 650 mV

pH = 10

pH

M₁    Meßstrom: 100 l/h    M₂

Gichtgaswaschwasser

vom Hochofen

Ringstrom: 2 m³/h

Klärbecken

Sammelbecken

Hauptstrom: 1500 m³/h

Kanal

0 068 458